# EUROPEAN PATENT SPECIFICATION

(11) **EP 1 864 673 B1**
(45) Date of publication and mention of the grant of the patent: **07.08.2013**
(21) Application number: 06733213.0
(22) Date of filing: 17.02.2006
(51) Int. Cl.: A61K 35/10, A61K 33/24, A61K 41/00, A61P 35/00

(54) **METHOD FOR PRODUCING AN ANTI-CANCER AGENT**
VERFAHREN ZUR HERSTELLUNG EINES KREBSMITTELS
PROCEDE DE FABRICATION D'UN PRODUIT ANTICANCEREUX

(30) Priority: 17.02.2005 RU 2005104288
(43) Date of publication of application: 12.12.2007
(73) Proprietor: Rdinnovation APS, 1360 (DK)
(72) Inventor: SHIPOV, Valery Pavlovich, ST. Petersburg, 194156 (RU); PIGAREV, Evgeny Sergeevich, St. Petersburg, 193967 (RU); FEDOROS, Elena Ivanovna, St. Petersburg, 193315 (RU); TROFIMOVA, Nadezhda Petrovna, St. Petersburg, 194156 (RU)
(74) Representative: Scholz, Volker
(86) International application number: PCT/RU2006/000077
(87) International publication number: WO 2006/088393

(56) References cited:
- EP-A2- 0 281 463
- KR-A1- 940 010 295
- RU-C1- 2 086 559
- RU-C1- 2 182 482

## Description

### Field of invention

The invention relates to a method for producing pharmaceuticals, and in particular, to producing of anti-cancer agent in the form of coordination compounds of metals; more precisely - to a method for producing of anti-cancer agent containing platinum complexes.

### Prior art

Antineoplastic and anticancer drugs containing coordination compounds of platinum cause damage to the genetic apparatus of tumor cells, and exhibit strong antineoplastic activity, thereby attracting attention of the specialists.

Previously known is a method for production of a coordination compound, *cis-*dichloroammineisopropylammineplatinum (II) from an aqueous solution of potassium tetrachloroplatinate (II) using organic ligands, ammonia and isopropylamine (A, Russian Federation, 2086559). Under the method, the solution of potassium tetrachloroplatinate (II) is subjected to the interaction with ammonium oxalate and then consecutively treated with solutions of ammonia, isopropylamine and hydrochloric acid.

A description accompanying the title of protection contains an example illustrating the claimed method for production of platinum coordination compound. Theoretically calculated quantities of potassium tetrachloroplatinate K₂[PtCl₄] and oxalate ion in the form of potassium oxalate K₂C₂O₄·H₂O and ammonium oxalate (NH₄)₂C₂O₄ are dissolved in the calculated volume of water and heated to 60°C for 30 minutes, maintaining pH=6.5 by adding sodium hydroxide until the solution becomes orange in color. Then, the solution is cooled down to 25°C and the required quantity of isopropylamine is added. The reaction mixture is held for 2 hours, not allowing the solution to darken. Then, a calculated quantity of concentrated hydrochloric acid is added, and the mixture is held for 40 minutes. The yellow precipitate of *cis-*dichloroammineisopropylammineplatinum (II) is then filtered, rinsed with alcohol and dried. The total yield of the complex evolved is 47% of theoretical.

The shortcomings of this method include relatively poor economic efficiency caused by the fact that platinum compound exhibit a very low reactivity with organic compounds, and, for a ratio of about 1:1 between the potential ligands and platinum compounds in the reaction solution under the known method, a substantial fraction of the platinum compound fails to form the target product.

Also previously known is a method for production of an antineoplastic drug (A, Russian Federation, 2182482) based on a coordination compound of platinum (II), namely potassium tetrachloroplatinate. Under the method, an aqueous solution of humic substances is treated with a solution of potassium tetrachloroplatinate. The treatment is conducted under irradiation with ultrasonic waves with the power density of 40 W/cm² and the frequency of 22 kHz for 4 to 8 minutes.

As indicated in the description, humic substances are obtained by oxidation of wood lignin in alkaline environment by oxygen-containing gas at the temperature of 170±20°C and pressure of 1.9-2.5 MPa for 1 to 3 hours, with subsequent cooling of the reaction mix, separation of the solid phase from the solution and acidification of the solution to pH=2...3. The precipitate formed after acidification of the solution is purified and dissolved in distilled water.

The solution of humic substances thus obtained (namely, an aqueous solution of salts of humic acids) is doped with a calculated quantity of potassium tetrachloroplatinate, and massive acoustic cavitation is induced by exposure to ultrasound with the power density of 40 W/cm³ and frequency of 22 kHz for 4 minutes.

The product obtained is an aqueous solution of salts of coordination compounds of platinum, with fragments of humic substances acting as ligands. The solution was used fir animal testing, the results of which confirmed its antineoplastic activity.

This method allows for a fuller utilization of platinum compounds, as the ratio of the source platinum compounds to organic (humic) substances is several times lower than in the above method, i.e. there are approximately three times more potential ligands per unit (molecule) of the platinum compound.

The antineoplastic (including anticancer) activity of the product obtained is due to the content of platinum and the properties of humic substances. The product has a low toxicity due to a relatively low concentration of platinum compounds. The treatment with ultrasound with the parameters indicated provides for a fairly efficient interaction of potassium tetrachloroplatinate with humic substances, which form polynuclear chelate complexes exhibiting antineoplastic activity.

It has been shown experimentally that the most active among humic substances are water-soluble substances with the molecular weight in the 500-10000 dalton range. Components of higher molecular weight are less active because of low solubility and very poor uptake by the body.

In concentrated (over 0.5-1%) solutions, humic substances undergo frequent intermolecular interactions, namely the reactions of condensation and polymerization, which lead to an increase in the average molecular weight. Therefore, in order to ensure efficient interaction and production of complexes with platinum compounds, the concentration of humic substances in the solution should be relatively low. However, even in these conditions, some molecules of humic substances participate in condensation and polymerization reactions with each other, forming conglomerates, or compounds with a higher molecular weight.

Besides the impairment of antineoplastic activity of such preparations, it leads to an impaired homogeneity of the products obtained, precipitate fallout, changes in physico-chemical properties, and instability of the preparations in storage. Thus, when stored under artificial aging conditions for as short a period as 10-20 days, the preparation obtained by the known method exhibited the presence of foreign inclusions (high molecular weight compounds) and color changes attesting to degradation of physico-chemical properties of the preparation.

The present invention is based upon the problem of developing a method for producing an anti-cancer agent allowing to produce a more homogeneous preparation with a better stability in storage by reducing the relative content of high molecular weight compounds.

This problem is solved by the method for producing an anti-cancer agent as defined in claim 1 based on a coordination compound of platinum (II), involving the treatment of an aqueous solution of humic compounds with the said platinum compound, under which the treatment is conducted under irradiation with waves; according to the invention, the processing is continued until the fraction of high molecular fraction humic compounds is brought to a level of 5% or less.

It has been established experimentally that the reduction of high molecular weight fraction below the said level allows for a substantial improvement of preparation homogeneity and its stability in storage.

The coordination compounds of platinum may be selected from the following range: potassium tetrachloroplatinate, *cis*-dichlorodiammineplatinum, hydrogen tetrachloroplatinate, potassium tetrabromoplatinate.

All these substances are compounds of platinum (II), which are used for production of antineoplastic and/or anticancer preparations.

Waves for irradiation may be reasonably selected in the ultrasonic range, with frequencies from 18 kHz to 66 kHz and power density from 0.5 to 5 W/cm³.

The preferred treatment procedure involves the irradiation power density of 5 W/cm³ at the frequency of 22 kHz for 5 to 20 minutes.

Irradiation may also be performed with microwaves in the frequency range from 0.3 to 30 GHz and power from 0.5 to 50 kW. For 5 W/cm³ irradiation at the frequency of 2.45 GHz, the product being treated is maintained at the temperature of 60-70°C for 30 to 90 minutes.

### Brief description of the drawings

The invention is illustrated by the drawings, on which:
Fig. 1 shows the dependence of the properties of humic substances in the preparation on the duration of ultrasonic exposure at the power density of 0.5 W/cm² and the frequency of 22 kHz.
Fig. 2 shows the dependence of the properties of humic substances in the preparation on the duration of ultrasonic exposure at the power density of 5 W/cm² and the frequency of 66 kHz.

### Preferred embodiment of the invention

The method claimed is described in more detail in the examples provided below.

Humic substances for production of anti-cancer agent were obtained by the method provided in (A, Russian Federation, 2182482) and described above.

In all the examples given below, the quantity of the platinum coordination compound has been selected to match the platinum content in potassium tetrachloroplatinate, i.e. the quantity of platinum compound used per 1 gram of the salts of humic acids corresponded to platinum content of 0.1242 g.

### Example 1.

Preparation 1 is obtained by adding 0.27 g potassium tetrachloroplatinate (a coordination compound of platinum) to 1 g salts of humic acids under acoustic cavitation caused by exposure to ultrasound with the power density of 40 W/cm² and the frequency of 22 kHz for 4 minutes.

Preparation 2 differs from preparation 1 by the use of dichlorodiammineplatinum as the platinum coordination compound, in the amount of 0.18 g per 1 g of humic salts.

Preparation 3 contains potassium tetrabromoplatinate as the platinum coordination compound in the amount of 0.40 g per 1 g of humic salts.

Preparation 4 contains hydrogen tetrachloroplatinate as the platinum coordination compound in the amount of 0.22 g per 1 g of humic salts.

The anticancer activity of the preparations was investigated on the RA-23 rhabdomyosarcoma model, typically used for the study of ability of antineoplastic and anticancer preparations to inhibit the development of stroma typical for most tumors.

The experiment used mongrel rats divided into five groups. The preparations were administered in the dose of 0.8 ml per rat every third day, starting from the day following the tumor inoculation. The control group received injections of normal saline solution. On the 20^{th} day, the animals were sacrificed and the number and weight of metastases were determined by autopsy. Statistical processing of the data was conducted using Student's *t*-criterion.

The results of the experiment are presented in Table 1. All the preparations produced exhibited roughly equal powerful anticancer activity, predominantly resulting in a decrease of the weight of metastases.

The results of comparative research of anticancer preparations containing different coordination compounds of platinum are presented in Table 1. Table 1.

| Metastatic index and growth of metastases | Group 1 (control) | Anticancer preparations | | | |
|---|---|---|---|---|---|
| | | Group 2 (preparation with potassium tetrachloroplatinate) | Group 3 (preparation with dichlorodiammin eplatinum) | Group 4 (preparation with potassium tetrabromoplatinate) | Group 5 (preparation with hydrogen tetrachloroplatinate) |
| Number of animals in the group | 19 | 19 | 20 | 18 | 20 |
| Number of metastases | 595 | 579 | 605 | 548 | 612 |
| Average number of metastases per animal | 31.3±6.8 | 30.5±9.5 | 30,25±9.3 | 30.7±9.4 | 30.6±7.9 |
| Average weight of one metastasis (mg) | 50.7±2.6 | 37.2±3.3 | 38.4±3.5 | 37.8±3.8 | 41.5±2.9 |
| % to the control group | 100 | 73.4 | 75.7 | 74.5 | 81.8 |

### Example 2.

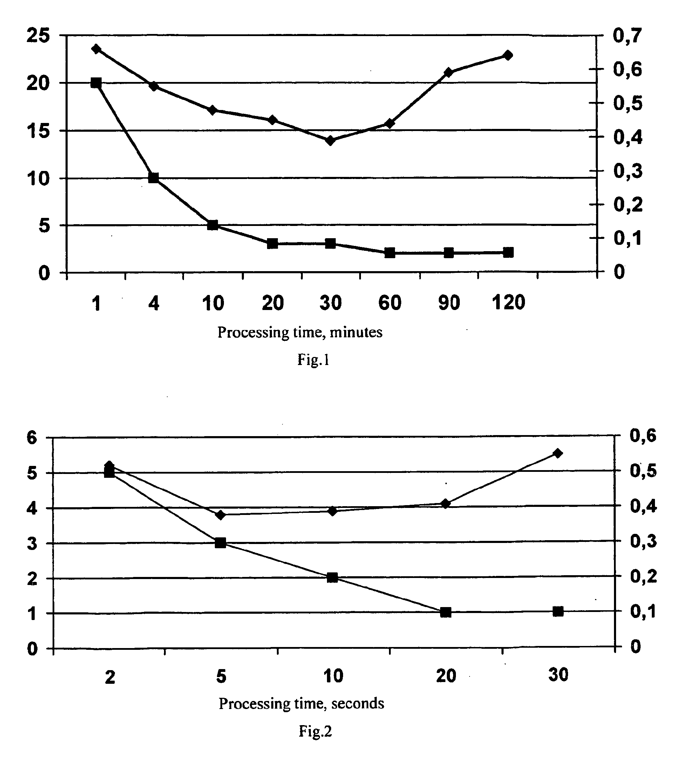

The preparations for research were produced in the same manner as the Preparation 1 in Example 1, except for the duration of ultrasonic exposure. The exposure time varied from 1 to 180 minutes. The preparations for checked for their color index (D₄₄₅ / D₆₆₅), the changes in which indirectly attest to the progress of molecular condensation and polymerization processes, and for molecular weight. The color index was measured for 0.001% standardized concentration solutions using a spectrophotometer with a 10 mm cell. Molecular weight was assessed by means of thin-layer chromatography on Silufol plates by Chemapol (Czech Republic) using the standard eluent in the 1:4 concentration. The level of high molecular weight fraction (X, %) was assessed by comparing the color intensity of the starting spot with the residual spot after elution. The dependence of properties of humic substances in the preparations on the duration of ultrasonic exposure at the power density of 0.5 W/cm³ and the frequency of 22 kHz is shown in Fig. 1, where the horizontal axis indicates the exposure time in minutes, the left vertical axis - the fraction of high molecular weight humic subtances (in %), and the right vertical axis - the color index. Curve 1 shows the fraction of high molecular weight humic substances as a function of exposure time, curve 2 - the color index as a function of exposure time. One can see from Fig. 1 that as the ultrasonic exposure time for the given wave irradiation parameters increases, the fraction of high molecular weight humic substances systematically decreased from 20% to 2%, whereas the color index first drops from 0.66 to 0.39 in the interval from 1 to 30 minutes, then increases again to 0.63. Thus, the best dispersal effect for the given frequency and power is achieved in the exposure time range from 10 to 30 minutes.

### Example 3.

The preparation was produced in a manner similar to the one described in Example 2, except for the ultrasonic treatment regimes. The treatment was conducted with the power density of 5 W/cm³ and the frequency of 66 kHz. The exposure time varied from 2 to 30 seconds. The fraction of high molecular weight humic substances (X, %) and the color index (D4/D6) were assessed for the preparations obtained as described in Example 2. The dependence of the properties of humic substances in the preparation on the duration of ultrasonic exposure at the power density of 5 W/cm² and the frequency of 66 kHz is presented in Fig. 2. Curve 3 illustrates the dependence of the fraction of high molecular weight humic substances (X, %) on the exposure time, curve 4 - the dependence of D4/D6 color index of the solution on the exposure time (in seconds) for the given wave irradiation parameters.

As the ultrasonic exposure times at the frequency of 66 kHz and power density of 5 W/cm³ increases, the fraction of high molecular weight humic substances systematically decreases, with the optimal values of the color index being observed for exposure times from 5 to 20 seconds.

The result of experiments conducted have demonstrated similar results to be achieved under different regimes of ultrasonic treatment, with the exposure time decreasing with the increase of ultrasound power and frequency.

### Example 4.

The preparation was produced in a manner similar to the one described in Examples 1 and 2, except for the wave treatment. In this case, dispersal was achieved by microwave treatment at the power of 0.5 kW and frequency of 2.45 GHz, with the temperature maintained in the range of 40-50°C.

The effect of microwave exposure time (for the power of 0.5 kW and the frequency of 2.45 GHz) upon the properties of the preparation is presented in Table 2.

**Table 2.**

| | Exposure time, minutes | | | | | | | | |
|---|---|---|---|---|---|---|---|---|---|
| | 10 | 20 | 30 | 60 | 90 | 120 | 180 | 240 | 300 |
| D₄₄₅/D_{66 5} | 0.67 | 0.55 | 0.48 | 0.46 | 0.42 | 0.45 | 0.57 | 0.60 | 0.65 |
| X, % | 15 | 10 | 7 | 5 | 3 | 3 | 2 | 2 | 2 |

It can be seen from the table that microwave treatment of the preparation for 60-120 minutes provides for the best characteristics of the product in terms of color index and fraction of high molecular weight humic substances, and may be used in lieu of ultrasonic treatment.

### Example 5.

Stability of the preparations produced as described in Examples 2-4 and having the optimal molecular weight and color index parameters was studied in "artificial aging" experiments, which involved storage of the preparations at the temperature of 60°C for 45 days. The stability criteria included pH, the content of molecular aggregates (appearing as foreign inclusions under fluorimetric analysis) and changes in the color index. The principal results are presented below.

The changes in pH of the solutions of preparations produced under different variants of the method claimed when stored under "accelerated aging" conditions are presented in Table 3.

The treatment regimes are abbreviated in the table: US for ultrasonic treatment, MW for microwave treatment.

**Table 3**

| pH of a solution of the preparation in the course of storage | | | | | |
|---|---|---|---|---|---|
| Preparation, production method | Initial solution | 10 days | 20 days | 30 days | 45 days |
| US 22 kHz 10 minutes | 8.71 | 8.80 | 8.81 | 8.83 | 8.78 |
| US 22 kHz 60 minutes | 8.79 | 8.80 | 8.81 | 8.78 | 8.78 |
| US 66 kHz 5 seconds | 8.82 | 8.82 | 8.81 | 8.81 | 8.80 |
| US 66 kHz 20 seconds | 8.82 | 8.82 | 8.80 | 8.81 | 8.80 |
| MW 2.45 GHz 60 minutes | 8.70 | 8.72 | 8.70 | 8.71 | 8.77 |
| MW 2.45 GHz 120 minutes | 8.80 | 8.80 | 8.80 | 8.89 | 8.78 |
| Prototype | 8.74 | 8.79 | 8.70 | 8.64 | 8.61 |

The number of specimens revealing foreign inclusions under fluorimetric analysis (in a series of 100 specimens) in the course of storage under "accelerated aging" conditions of the preparation specimens produced under different variants of the method claimed is shown in Table 4.

**Table 4.**

| Number of specimens with foreign inclusions (in a series of 100 specimens) | | | | | |
|---|---|---|---|---|---|
| Preparation, production method | Initial solution | 10 days | 20 days | 30 days | 45 days |
| US 22 kHz 10 minutes | None | None | None | 1 | 1 |
| US 22 kHz 60 minutes | None | None | None | None | None |
| US 66 kHz 5 seconds | None | None | None | 1 | 1 |
| US 66 kHz 20 seconds | None | None | None | None | None |
| MW 2.45 GHz 60 minutes | None | None | None | None | 1 |
| MW 2.45 GHz 120 minutes | None | None | None | None | None |
| Prototype | 1 | 1 | 2 | 2 | 3 |

The changes in color index of preparation specimens produced by different variants of the method in the course of storage under "accelerated aging" conditions are presented in Table 5.

**Table 5.**

| D₄₄₅/D₆₆₅ | | | | | |
|---|---|---|---|---|---|
| Preparation, production method | Initial solution | 10 days | 20 days | 30 days | 45 days |
| US 22 kHz 10 minutes | 0.48 | 0.50 | 0.51 | 0.49 | 0.52 |
| US 22 kHz 60 minutes | 0.44 | 0.43 | 0.46 | 0.48 | 0.48 |
| US 66 kHz 5 seconds | 0.38 | 0.42 | 0.41 | 0.43 | 0.42 |
| US 66 kHz 20 seconds | 0.41 | 0.44 | 0.45 | 0.43 | 0.46 |
| MW 2.45 GHz 60 minutes | 0.46 | 0.48 | 0.51 | 0.53 | 0.49 |
| MW 2.45 GHz 120 minutes | 0.45 | 0.49 | 0.50 | 0.52 | 0.50 |
| Prototype | 0.55 | 0.60 | 0.65 | 0.67 | 0.70 |

The "accelerated aging" test has demonstrated the preparations produced according to the invention to exhibit better stability characteristics (namely, the color index and the occurrence of foreign inclusion) at the end of the accelerated aging period, compared to the prototype. The acidity figures remained approximately the same for the specimens produced by the method claimed and those produced by the prototype method.

### Industrial applicability

The method proposed allows to produce preparations exhibiting anticancer activity having better stability characteristics in comparison with the prior method.

The method proposed may be implemented using standard equipment and the usual raw materials.

## Claims

1. A method for producing an anti-cancer agent based on a coordination compound of platinum (II), according to which the given platinum compound is used to treat an aqueous solution of humic compounds while under irradiation with waves, **characterized in that** the treatment is continued until the fraction of the humic compounds with a molecular weight above 10,000 Daltons is brought to a level of 5% or less.

2. A method for producing an anti-cancer agent according to claim 1, **characterized in that** the coordination compounds of platinum are selected from the following range: potassium tetrachloroplatinate, cis-dichlorodiamineplatinum, hydrogen tetrachloroplatinate, potassium tetrabromoplatinate.

3. A method for producing an anti-cancer agent according to claims 1 or 2, **characterized in that** the wave radiation is selected fin the ultrasonic frequency range from 18 kHz to 66 kHz, with the irradiation power density in the range of 0.5 to 5 W/cm³.

4. A method for producing an anti-cancer agent according to claim 3, **characterized in that** the treatment is conducted at the irradiation power density of 5 W/cm³ at the frequency of 22 kHz for 5 to 20 minutes.

5. A method for producing an anti-cancer agent according to claims 1 to 2, **characterized in that** the wave radiation is selected in the microwave frequency range from 0.3 kHz to 30 GHz, with the irradiation power in the range of 0.5 to 50 kW.

6. A method for producing an anti-cancer agent according to claim 4, **characterized in that** the treatment is conducted at the irradiation power density of 5 W/cm³ at the frequency of 2.45 kHz, with the temperature of the product under treatment being maintained in the range of 60-70°C for 30 to 90 minutes.

## Patentansprüche

1. Verfahren zum Herstellen eines Anti-Krebsmittels basierend auf einer Koordinationsverbindung von Platin(II), gemäß welchem die gegebene Platinverbindung verwendet wird, um eine wässrige Lösung von humitischen Verbindungen, während diese unter Bestrahlung mit Wellen ist, zu behandeln, **dadurch gekennzeichnet, dass** die Behandlung fortgeführt wird, bis die Fraktion der humitischen Verbindungen mit einem Molekulargewicht oberhalb von 10.000 Dalton auf ein Niveau von 5% oder weniger gebracht ist.

2. Verfahren zum Herstellen eines Anti-Krebsmittels nach Anspruch 1, **dadurch gekennzeichnet, dass** die Koordinationsverbindungen von Platin ausgewählt werden aus dem folgenden Bereich: Kaliumtetrachloroplatinat, cis-Dichlorodiaminplatin, Wasserstofftetrachloroplatinat, Kaliumtetrabromoplatinat.

3. Verfahren zum Herstellen eines Anti-Krebsmittels nach Anspruch 1 oder 2, **dadurch gekennzeichnet, dass** die Wellenstrahlung ausgewählt wird im Ultraschallfrequenzbereich von 18 kHz bis 66 kHz, wobei die Bestrahlungsleistungsdichte im Bereich von 0,5 bis 5 W/cm³ liegt.

4. Verfahren zum Herstellen eines Anti-Krebsmittels nach Anspruch 3, **dadurch gekennzeichnet, dass** die Behandlung bei der Bestrahlungsleistungsdichte von 5 W/cm³ bei der Frequenz von 22 kHz für 5 bis 20 Minuten durchgeführt wird.

5. Verfahren zum Herstellen eines Anti-Krebsmittels Anspruch 1 oder 2, **dadurch gekennzeichnet, dass** die Wellenstrahlung ausgewählt wird im Mikrowellenfrequenzbereich von 0,3 kHz bis 30 GHz, wobei die Bestrahlungsleistung im Bereich von 0,5 bis 50 kW liegt.

6. Verfahren zum Herstellen eines Anti-Krebsmittels nach Anspruch 4, **dadurch gekennzeichnet, dass** die Behandlung bei der Bestrahlungsleistungsdichte von 5 W/cm³ bei der Frequenz von 2,45 kHz durchgeführt wird, wobei die Temperatur des Produkts unter Behandlung im Bereich von 60-70°C für 30 bis 90 Minuten gehalten wird.

## Revendications

1. Procédé de production d'un agent anticancéreux sur la base d'un composé de coordination de platine (II), selon lequel le composé de platine donné est utilisé pour traiter une solution aqueuse de composés humiques tout en étant sous irradiation par ondes, **caractérisé en ce que** le traitement est maintenu jusqu'à ce que la fraction des composés humiques avec une masse moléculaire au-dessus de 10000 Daltons soit amenée à un niveau de 5% ou moins.

2. Procédé de production d'un agent anticancéreux selon la revendication 1, **caractérisé en ce que** les composés de coordination de platine sont choisis parmi la gamme suivante : tétrachloroplatinate de potassium, cis-dichlorodiamineplatine, tétrachloroplatinate d'hydrogène, tétrabromoplatinate de potassium.

3. Procédé de production d'un agent anticancéreux selon la revendication 1 ou 2, **caractérisé en ce que** le rayonnement d'ondes est choisi dans la gamme des fréquences ultrasonores de 18 kHz à 66 kHz, la densité de puissance d'irradiation étant dans la plage de 0,5 à 5 W/cm3.

4. Procédé de production d'un agent anticancéreux selon la revendication 3, **caractérisé en ce que** le traitement est conduit au niveau de la densité de puissance d'irradiation de 5 W/cm³ au niveau de la fréquence de 22 kHz. pendant 5 à 20 minutes.

5. Procédé de production d'un agent anticancéreux selon la revendication 1 à 2, **caractérisé en ce que** le rayonnement d'ondes est choisi dans la gamme des fréquences microondes de 0,3 kHz à 30 GHz, la puissance d'irradiation étant dans la plage de 0,5 à 50 kW.

6. Procédé de production d'un agent anticancéreux selon la revendication 4, **caractérisé en ce que** le traitement est conduit au niveau de la densité de puissance d'irradiation de 5 W/cm³ au niveau de la fréquence de 2,45 kHz, la température du produit sous traitement étant maintenue dans la plage de 60-70 °C pendant 30 à 90 minutes.
